# FASCICULE DE BREVET EUROPEEN

(11) **EP 3 057 560 B1**
(45) Date de publication et mention de la délivrance du brevet: **30.12.2020**
(21) Numéro de dépôt: 14784473.2
(22) Date de dépôt: 16.10.2014
(51) Int. Cl.: A61J 1/20

(54) **DISPOSITIF D'INTERFAÇAGE D'UN FLACON À PERFORER**
VORRICHTUNG ZUR SCHNITTSTELLENBILDUNG MIT EINER ZU DURCHSTECHENDEN AMPULLE
DEVICE FOR INTERFACING A FLASK TO BE PERFORATED

(30) Priorité: 16.10.2013 FR 1360084
(43) Date de publication de la demande: 24.08.2016
(73) Titulaire: Vygon, 95440 Ecouen (FR)
(72) Inventeur: CARREZ, Jean-Luc, F-95440 Ecouen (FR); COUSSEGAL, Jean-Louis, F-95250 Beauchamp (FR); BARRE, Laurent, F-95440 Ecouen (FR); ALGRAIN, Isabelle, F-95380 Puiseux en France (FR)
(74) Mandataire: Regimbeau
(86) Numéro de dépôt international: PCT/EP2014/072181
(87) Numéro de publication internationale: WO 2015/055751

(56) Documents cités:
- EP-A2- 0 126 718
- WO-A1-97/20536
- WO-A2-2007/120641
- FR-A1- 2 951 638
- US-A1- 2007 032 775
- US-A1- 2012 078 179

## Description

### DOMAINE DE L'INVENTION

L'invention concerne un dispositif d'interfaçage destiné à mettre en liaison un instrument d'injection de fluide tel qu'une seringue et un flacon à perforer contenant un médicament à des fins de préparations de fluides destinés à être perfusés chez un patient.

### ARRIERE-PLAN TECHNOLOGIQUE

En oncologie, pour le traitement des tumeurs cancéreuses, il est nécessaire de perfuser des fluides pouvant être très agressifs. La préparation de ces fluides, qui se présentent sous forme de soluté, peut s'avérer dangereuse pour le personnel soignant dans la mesure où elles sont très agressives et peuvent être la source d'accidents avec des effets toxiques ou allergiques. Dans beaucoup de cas, le médicament qui doit être administré se présente sous la forme de poudre dans un flacon sous vide, qui est obturé par un bouchon à membrane élastomérique.

Afin de mélanger ce médicament contenu dans un tel flacon sous vide à un fluide destiné à être perfusé, il faut dans un premier temps diluer le médicament sous forme de poudre dans ce liquide à l'aide d'une seringue généralement équipée d'une aiguille. Le fluide à perfuser peut par exemple être du sérum physiologique (eau distillée + NaCl).

On traverse alors le bouchon à membrane élastomérique du flacon avec l'aiguille, ce qui a pour effet de faire entrer de l'air brutalement dans le flacon qui était sous vide. Cet air se mélange avec le médicament sous forme de poudre et peut ressortir en partie en entrainant du médicament pur, ce qui est dangereux pour le personnel soignant chargé de la préparation. Une fois l'aiguille de la seringue mise en place à travers le bouchon à membrane élastomérique, le personnel soignant chargé de la préparation pousse à l'aide de la seringue le fluide à perfuser dans le flacon, ce qui fait ressortir, là encore, de l'air enfermé initialement dans le flacon chargé le cas échéant de particules de médicaments non encore diluées.

Le flacon est alors secoué et le mélange est aspiré à travers le bouchon à membrane élastomérique à l'aide de la seringue. En pratique, le mélange liquide à perfuser plus médicament dilué est injecté et aspiré plusieurs fois de suite hors et dans le flacon avec la seringue, afin de bien mélanger le médicament dans le liquide. Cependant, un tel mélange provoque des entrées et des sorties d'air du le flacon, ce qui implique des risques de pollution d'une part pour le personnel soignant chargé de la préparation, et d'autre part pour le mélange préparé.

Enfin, l'aiguille est sortie du flacon. Il faut alors la déconnecter de la seringue sans se piquer et éviter tout contact direct du mélange ainsi réalisé avec le personnel soignant chargé de la préparation. La seringue remplie est alors connectée près du patient à une poche de perfusion reliée à celui-ci ou connectée directement au patient lui-même.

Au cours de cette manipulation, les principales causes d'accident sont liées à des problèmes d'étanchéité aux liquides et aux gaz de l'intérieur du flacon vers le milieu extérieur, avec dissémination de produits dangereux, qui peuvent entrer en contact avec la peau, les yeux et le système respiratoire du personnel préparateur et soignant, mais également à des problèmes d'étanchéité du milieu extérieur vers l'intérieur du flacon qui risquent de contaminer la préparation, ou encore à des problèmes de stabilité verticale du dispositif d'interfaçage. Il peut donc s'avérer nécessaire que le personnel préparateur travaille dans des espaces confinés, sous hotte ou dans des enceintes, et avec des gants épais.

On connaît déjà de tels dispositifs d'interfaçage. Par exemple, le document FR 2 928 539 propose un dispositif d'interfaçage comprenant des moyens de circulation d'air, destinés à permettre des entrées et des sorties d'air du flacon à perforer, munis de moyens de filtration d'air adaptés pour retenir les particules de médicament pur non encore diluées lors de sortie d'air du flacon, ainsi que des impuretés contenues dans l'air ambiant pénétrant dans le flacon lors d'entrée d'air dans le flacon. Le dispositif permet donc de sécuriser la manipulation et la préparation du mélange.

Cependant, l'utilisation de ce dispositif entraine plusieurs entrées et sorties d'air dans le flacon qui posent encore des problèmes de sécurité. En effet, le moyen de filtration, qui est généralement sous la forme d'un filtre d'échappement, est très sensible aux liquides et son filtrage ne paraît pas suffisamment efficace pour les utilisateurs.

Par ailleurs, le dispositif est relativement complexe à réaliser et comprend un grand nombre de pièces.

On a donc proposé dans le document FR 2 951 648 un dispositif d'interfaçage comprenant une embase sur laquelle est fixée un réservoir d'air via des moyens de circulation d'air, dans lequel le réservoir d'air comprend une coque rigide associée à une membrane souple, définissant un compartiment isolé de l'extérieur. Ce système de circulation d'air amélioré remplace alors le filtre et permet une circulation d'air dans le flacon, tout en maintenant le flacon isolé de l'extérieur et donc des risques de contamination par des éléments extérieurs.

Un tel dispositif d'interfaçage présente de bonnes fonctionnalités et permet de limiter efficacement les risques de contamination. Toutefois, sa conception est très coûteuse et complexe. Par ailleurs, cette conception ne permet pas de prélever l'intégralité des fluides dans le flacon.

Par la suite a été proposé dans le document FR 2 982 484 un dispositif d'interfaçage plus stable dans toutes les positions et au cours des manipulations. Ce dispositif conserve le système fermé de circulation d'air mais propose un autre moyen de fixation de l'embase au flacon au travers d'un accrochage type harpon plutôt qu'un bouchon encliquetable. Pour cela, FR 2 982 484 propose un dispositif d'interfaçage destiné à mettre en liaison un instrument d'injection d'un fluide et un flacon à perforer, comprenant :
- une embase adaptée pour connecter le dispositif d'interfaçage à l'instrument d'injection du fluide,
- un moyen de fixation de l'embase sur le flacon à perforer, agencé au niveau d'une extrémité distale du dispositif d'interfaçage, présentant une extrémité proximale divergente formant un épaulement et une extrémité distale convergente adaptée pour faciliter une pénétration du moyen de fixation dans le flacon à perforer, dans lequel le moyen de fixation comprend au moins une échancrure dans une zone adjacente à son extrémité proximale adaptée pour mettre en communication fluidique le flacon à perforer avec un espace externe au dispositif d'interfaçage.

Ce dispositif améliore nettement l'ergonomie du dispositif. Toutefois, sa conception reste relativement coûteuse et complexe.

Il a également été proposé dans le document WO 2007/120641 un dispositif d'interfaçage comprenant une embase configurée pour être connectée d'une part à un instrument d'injection et d'autre part à un flacon à perforer. Toutefois, le dispositif décrit dans ce document est très complexe à mettre en œuvre, dans la mesure où il propose d'introduire un sachet dans le flacon lors de la perforation afin de compenser les différences de pression. La présence d'un tel sachet dans le flacon risque cependant de gêner l'opérateur lors des aspirations et réinjections successives pour mélanger le contenu du flacon avec le fluide, d'empêcher le prélèvement complet du fluide et éventuellement de présenter des problèmes d'étanchéité.

Le document WO 97/20536 quant à lui décrit un dispositif pour la préparation d'une solution médicamenteuse reconstituée à partir de deux composants. Toutefois, dans ce document, les différences de pression sont compensées à l'aide d'un filtre à particules qui, comme nous l'avons vu plus haut, est très sensible aux liquides et n'est pas suffisamment efficace aux yeux des utilisateurs.

### RESUME DE L'INVENTION

Un objectif de l'invention est donc de proposer un dispositif d'interfaçage qui soit ergonomique pour un opérateur, facilite les manipulations de fixation, de mélange et d'aspiration de fluide, notamment dans le domaine de l'oncologie, tout en limitant les risques de contamination de l'opérateur, que ce soit du flacon vers l'extérieur ou de l'extérieur vers le flacon et en permettant de prélevé un plus grand volume de fluide quel que soit le volume du flacon à perforer.

Pour cela, l'invention propose un dispositif d'interfaçage destiné à mettre en liaison un instrument d'injection d'un fluide et un flacon à perforer, comprenant :
- une embase comprenant :
   * un premier conduit, adapté pour être fixé au flacon à perforer,
   * un deuxième conduit, adapté pour permettre la circulation des gaz dans le flacon à perforer, et
   * un troisième conduit, adapté pour être connecté à l'instrument d'injection du fluide,
- des moyens de circulation des fluides, comprenant un orifice proximal en communication fluidique avec le troisième conduit,
le dispositif d'interfaçage étant caractérisé en ce que les moyens de circulation des fluides comprennent en outre un tube, logé dans le deuxième conduit et mobile en translation dans ledit deuxième conduit, et comprenant au niveau de son extrémité distale un orifice distal en communication fluidique avec le deuxième conduit, l'orifice proximal et l'orifice distal étant configurés pour déboucher dans le flacon à perforer lorsque le dispositif d'interfaçage est fixé audit flacon à perforer.

Certaines caractéristiques préférées mais non limitatives de ce dispositif d'interfaçage sont les suivantes :
- les moyens de circulation des fluides comprennent un embout, fixé au niveau d'une extrémité distale du premier conduit de l'embase, ledit embout comprenant au niveau de son extrémité proximale l'orifice proximal,
- le tube est logé dans l'embout et est mobile en translation dans ledit embout,
- l'extrémité distale du tube est évasée,
- l'orifice proximal s'étend latéralement par rapport à une direction d'extension du tube,
- l'orifice proximal est formé au niveau d'une extrémité proximale de l'embout,
- une extrémité distale de l'embout est pointue,
- une extrémité proximale du tube est fixée sur un dispositif d'échange des gaz,
- le dispositif comprend en outre un manchon disposé dans le deuxième conduit et adapté pour maintenir le tube de façon étanche dans ledit deuxième conduit,
- le dispositif comprend en outre un organe de protection, fixé sur le premier conduit et adapté pour être fixé au flacon à perforer, et
- le premier conduit et le deuxième conduit sont coaxiaux, le troisième conduit formant un angle compris entre 30° et 60° avec le deuxième conduit.

### BREVE DESCRIPTION DES DESSINS

D'autres caractéristiques, buts et avantages de la présente invention apparaîtront mieux à la lecture de la description détaillée qui va suivre, et au regard des dessins annexés donnés à titre d'exemples non limitatifs et sur lesquels :
La figure 1 est une vue en coupe en position retournée d'une première forme de réalisation d'un dispositif d'interfaçage conforme à l'invention fixé sur un exemple de flacon à perforer, et sur lequel a été fixé un exemple de dispositif d'interfaçage,
La figure 2 représente une vue en coupe du dispositif d'interfaçage de la figure 1.
La figure 3 représente une vue en coupe du dispositif d'interfaçage de la figure 1, dans sa position d'introduction dans le flacon.
Les figures 4a et 4b représentent des vues en perspective de l'embout du dispositif d'interfaçage de la figure 1.
La figure 5 représente une vue en coupe de l'embase du dispositif d'interfaçage de la figure 1.
La figure 6 représente une vue en coupe du dispositif d'interfaçage de la figure 1, muni d'un organe de protection.
Les figures 7a et 7b représentent des vues en coupes de deux modes de réalisation de dispositif d'échange des gaz susceptibles d'être utilisé dans un dispositif d'interfaçage conforme à l'invention.

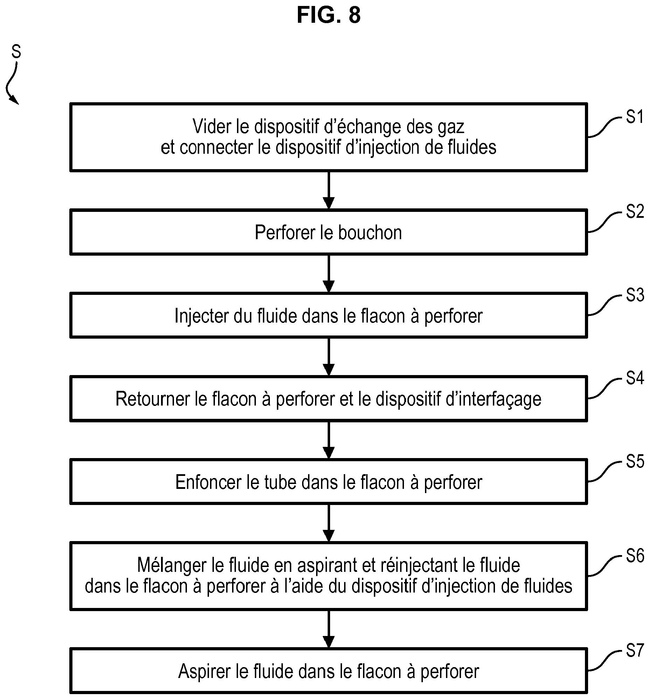

La figure 8 est un organigramme représentant différentes étapes d'un exemple de réalisation du procédé d'utilisation d'un dispositif d'interfaçage conforme à l'invention.

### DESCRIPTION DETAILLEE D'UN MODE DE REALISATION

Nous allons à présent décrire un dispositif d'interfaçage 1 en référence aux figures annexées.

Un dispositif d'interfaçage 1 destiné à mettre en liaison un instrument d'injection d'un fluide 2 et un flacon à perforer 3, comprend :
- une embase 20 comprenant :
   * un premier conduit 21, adapté pour être fixé au flacon à perforer 3,
   * un deuxième conduit 22, adapté pour permettre la circulation des gaz dans le flacon à perforer 3, et
   * un troisième conduit 23, adapté pour être connecté à l'instrument d'injection du fluide 2,
- des moyens de circulation des fluides 10, 40, configurés pour déboucher dans le flacon à perforer 3 lorsque le dispositif d'interfaçage 1 est fixé au flacon à perforer 3.

Les moyens de circulation 10, 40 des fluides comprennent un orifice distal 42, en communication fluidique avec le deuxième conduit 22, et un orifice proximal 11, en communication fluidique avec le troisième conduit 23, l'orifice proximal 11 et l'orifice distal 42 étant configurés pour déboucher dans le flacon à perforer 3 lorsque le dispositif d'interfaçage 1 est fixé audit flacon à perforer 3.

Le dispositif d'interfaçage 1 est destiné à être monté sur le flacon à perforer 3 au niveau d'une extrémité distale 21b du premier conduit 21 et sur l'instrument d'injection de fluide 2, comme par exemple une seringue (non représentée sur les figures), au niveau d'une extrémité proximale 23a du troisième conduit 23.

Tout moyen de connexion habituel entre l'instrument d'injection de fluide 2 et l'embase 20 peut être utilisé. De préférence, on choisira des moyens de connexion susceptibles de limiter les risques de formation de gouttes et facilement nettoyables, dans la mesure où les médicaments obtenus risquent d'être nocifs pour l'opérateur. Typiquement, l'instrument d'injection de fluide 2 pouvant être connecté à l'embase 3 peut comprendre une seringue sans aiguille, tel que le dispositif décrit dans le document FR 10 51136 au nom de la Demanderesse.

### L'embase

L'embase 20 comprend un corps en Y comprenant les premier 21, deuxième 22 et troisième 23 conduits.

Le premier conduit 21 s'étend selon un premier axe X1 principal de l'embase 20 et est adapté pour être relié de façon étanche au niveau de son extrémité distale 21b au flacon à perforer.

Le deuxième conduit 22 s'étend selon un deuxième axe X2 depuis une extrémité proximale 22a de l'embase 20 et est adapté pour permettre la circulation des gaz du flacon à perforer 3. Par exemple, un réservoir 31 peut être connecté au deuxième conduit 22 afin de former un circuit fermé pour le gaz contenu initialement dans le flacon à perforer 3 et/ou le réservoir 31.

Dans l'exemple de réalisation illustré sur les figures, le deuxième axe X2 et le premier axe X1 sont sensiblement parallèles. Par exemple, le deuxième conduit 22 peut s'étendre dans le prolongement du premier conduit 21, le premier axe X1 et le deuxième axe X2 étant alors confondus.

Par ailleurs, le premier conduit 21 et le deuxième conduit 22 sont en communication fluidique.

Le troisième conduit 23, quant à lui, s'étend selon un troisième axe X3 depuis l'embase 20 et comprend une extrémité proximale 23a adaptée pour recevoir le dispositif d'injection de fluide 2. Par exemple, l'extrémité proximale 23a peut comprendre des moyens de vissage de type Luer ou Luer Lock. La section interne du troisième conduit 23 peut notamment être réduite au niveau de son intersection 23b avec le premier 21 et/ou le deuxième 22 conduit, afin de faciliter l'injection et l'aspiration des fluides dans le flacon à perforer 3. Optionnellement, l'extrémité proximale 23a du troisième conduit 23 peut être obturée par une membrane (non illustrée sur les figures) pour étanchéifier le troisième conduit 23 tout en permettant la connexion d'un dispositif d'injection de fluide 2 tel qu'une seringue.

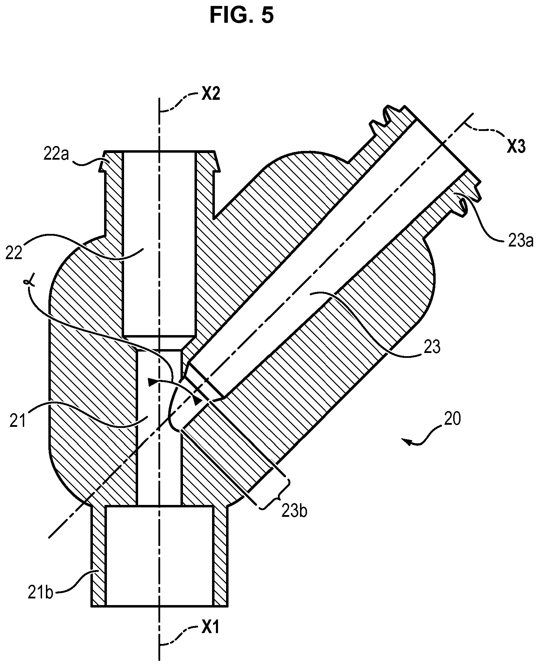

Le troisième axe X3 est de préférence incliné par rapport au premier axe X1 et au deuxième axe X2. Ainsi, dans l'exemple de réalisation illustré sur les figures, le troisième axe X3 forme un angle α compris entre 0° et 90° avec le deuxième axe X2, de préférence entre 30° et 60° (figure 5). Comme nous le verrons par la suite, cette inclinaison du troisième conduit 23 par rapport aux premier et deuxième conduits 21, 22 permet d'améliorer l'ergonomie du dispositif d'interfaçage 1 lors de l'introduction et l'aspiration de fluides dans le flacon à perforer 3.

### Les moyens de circulation des fluides

Les moyens de circulation des fluides 10, 40 sont adaptés pour permettre d'une part l'introduction et l'aspiration d'un fluide dans le flacon à perforer 3 à l'aide du dispositif d'injection de fluide 2, et d'autre part la circulation des gaz (entrées et sorties) du flacon à perforer 3.

A cet effet, les moyens de circulation des fluides comprennent un orifice distal 42, en communication fluidique avec le deuxième conduit 22, destiné à permettre la circulation des gaz du flacon à perforer 3, et un orifice proximal 11, en communication fluidique avec le troisième conduit 23, destiné à permettre l'injection et l'aspiration de fluide à l'aide du dispositif d'injection 2.

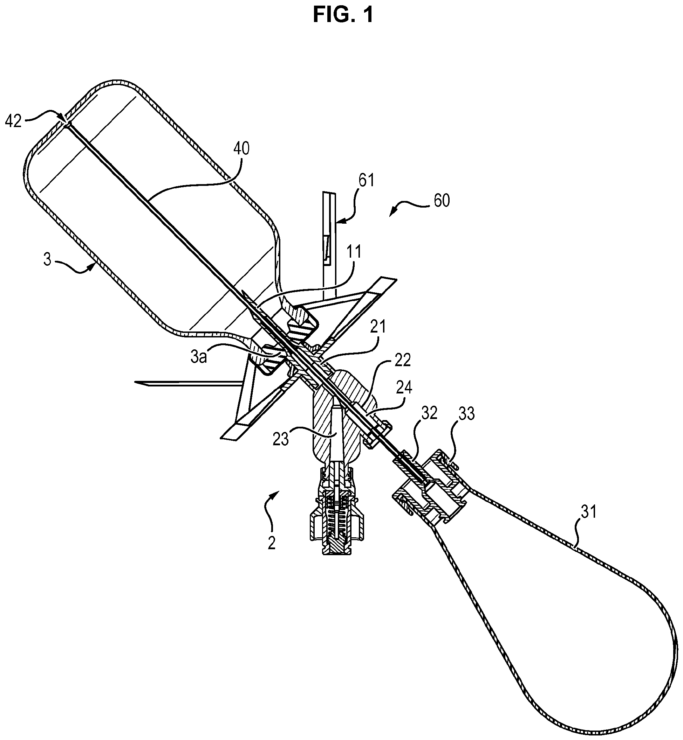

Avantageusement, le positionnement en partie distale de l'orifice 42 permettant la circulation des gaz et le positionnement en partie proximale de l'orifice 11 permettant la circulation des fluides (liquides) permettent d'améliorer nettement l'ergonomie et l'efficacité de l'injection et de l'aspiration des fluides dans le flacon à perforer 3. En effet, en utilisation, le flacon à perforer 3 et le dispositif d'interfaçage 1 sont retournés, de sorte que le flacon 3 se trouve au-dessus du dispositif d'interfaçage 1, comme illustré sur la figure 1. Les fluides étant plus denses que les gaz, ceux-ci descendent par conséquent vers le bouchon 3a de sorte que l'orifice distal 42, qui se situe en partie distale 40b des moyens de circulation des fluides, se trouve dans la région du flacon à perforer 3 comprenant les gaz, tandis que l'orifice proximal 11 est immergé dans le fluide.

Selon une forme de réalisation, les moyens de circulation des fluides 10, 40 comprennent un embout 10, fixé au niveau de l'extrémité proximale 21a du premier conduit 21 de l'embase 20, et un tube 40, logé dans le premier 21 et le deuxième 22 conduits de l'embase 20.

L'orifice proximal 11 des moyens de circulation des fluides est formé dans une extrémité proximale de l'embout 10, tandis que l'orifice distal 42 est formé au niveau d'une extrémité distale du tube 40.
L'embout 10 est configuré de sorte que, lorsque l'embase 20 est fixée sur le flacon à perforer 3, l'orifice proximal 11 se situe dans le volume interne du flacon à perforer 3. Par ailleurs, le tube 40 peut être mobile en translation dans les premier 21 et deuxième 22 conduits de l'embase 20, afin d'ajuster la position de l'orifice distal 42 en fonction du volume du flacon à perforer 3.

Ici, l'embout 10 est coaxial avec les premier 21 et deuxième 22 conduits, et est adapté pour recevoir le tube 40 de manière coulissante. Pour cela, l'embout 10 comprend un passage coaxial avec les premier et deuxième 22 conduits, ledit passage débouchant au niveau d'une extrémité distale 10b de l'embout 10 par un trou distal 12 à travers lequel le tube 40 peut faire saillie.

Dans la forme de réalisation illustrée sur les figures, l'embout 10 est de forme globalement cylindrique.

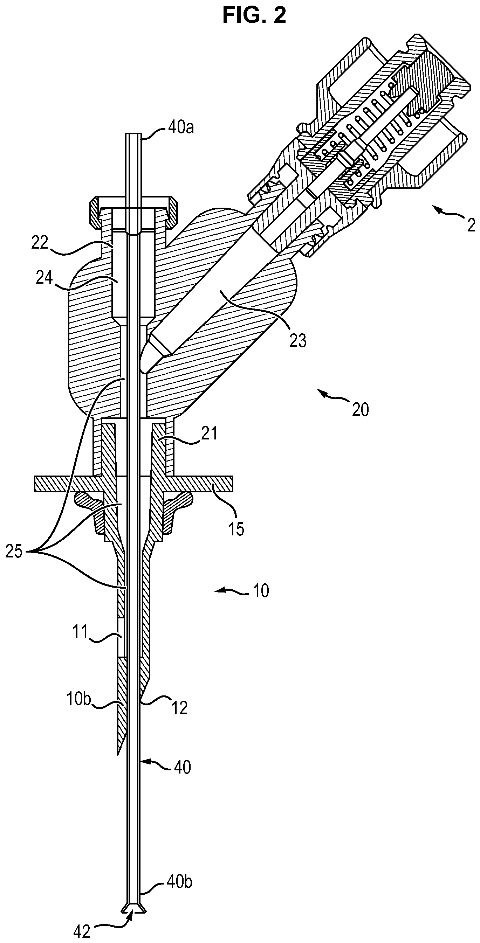

De préférence, le tube 40 est dimensionné de manière à former un espace 25 avec les parois internes du conduit 21 et de l'embout 10, de manière à permettre le passage des fluides entre l'orifice proximal 11 et le dispositif d'injection des fluides 2 (figure 2). Par exemple, ce passage peut s'étendre depuis l'orifice proximal 11, puis longer le tube 40 au sein de l'embout 10 et du premier conduit 21 pour déboucher dans le troisième conduit 23.

Avantageusement, l'espace ne communique pas avec le deuxième conduit 22, qui est dédié aux échanges gazeux avec le flacon à perforer 3 par l'intermédiaire du tube 40. Pour cela, le deuxième conduit 22 peut notamment être étanchéifié à l'aide d'un manchon 24, logé dans l'extrémité proximale 22a du deuxième conduit 22 et configuré pour permettre le passage et le mouvement en translation du tube 40 dans les premier 21 et deuxième 22 conduits, tout en empêchant le passage de fluides en provenance du dispositif d'injection de fluide 2 ou du flacon à perforer 3. Par exemple, le manchon 24 peut être de forme complémentaire de la paroi interne du deuxième conduit 22 et être traversé d'un trou central, coaxial avec les axes X1 et X2 des premier 21 et deuxième 22 conduits et configuré pour recevoir de façon étanche le tube 40. A cet effet, le manchon 24 peut notamment être réalisé dans un matériau élastomérique,

Dans l'exemple de réalisation illustré sur les figures, le deuxième conduit 22 est resserré en partie proximale 22a afin de former une butée pour le manchon 24 et le fixer en translation. Ainsi, l'espace 25 est étanchéifié au niveau du deuxième conduit 22 par le manchon 24 et le tube 40, déviant les fluides en provenance de l'orifice proximal 11 vers le troisième conduit 23.

On notera que, dans cette forme de réalisation, l'orifice proximal 11 peut être dans une paroi latérale de l'embout 10 et déboucher dans le passage 25. Avantageusement, l'orifice proximal 11 s'étend alors dans une zone très proche du bouchon 3a, ce qui augmente la quantité de fluides pouvant être absorbés.

En variante, le rôle de l'orifice proximal 11 pourrait être tenu par le trou distal 12 de l'embout 10, qui est traversé par le tube 40, l'orifice distal 42 restant au niveau de l'extrémité distale 40b du tube 40.

L'extrémité distale 40b du tube 40 peut présenter un évasement adapté pour empêcher l'extraction du tube 40 du flacon à perforer 3. Par exemple, lorsque le tube 40 est mobile en translation dans l'orifice distal 12 de l'embout 10, l'évasement est agencé pour venir en butée contre les parois formant l'orifice distal 12. Dans cette forme de réalisation, la prise de gaz peut donc se faire de l'orifice distal 42 de l'embout 10 jusqu'à la paroi de fond du flacon 3 (dans l'hypothèse où la longueur du tube 40 est suffisante pour atteindre cette paroi). Le gaz présent initialement dans le flacon à perforer 3 peut donc circuler librement et en circuit fermé, notamment lorsqu'un réservoir 31 est connecté de façon étanche au deuxième conduit 22.

Ainsi, l'orifice proximal 11 de l'embout 10, servant à la prise de fluide, se situe à proximité du bouchon 3a, alors que l'orifice distal 42 servant à la prise de gaz, peut se situer en tout point entre l'orifice distal 12 de l'embout 10 et le fond du flacon à perforer 3, lorsque le tube 40 est mobile en translation. Les prises d'air et de fluide sont donc décorrélées spatialement.

Le tube 40 peut notamment être en inox.

L'embout 10 peut être configuré pour perforer le flacon à perforer 3. A cet effet, l'extrémité distale 10b de l'embout 10, à travers laquelle passe le tube 40, peut être conformée de manière à traverser le bouchon 3a du flacon à perforer 3 de façon étanche. Typiquement, l'extrémité distale 10b de l'embout 10 peut être en forme de pointe.

En variante, la perforation peut être réalisée à l'aide du tube 40, d'un perforateur distinct de l'embout 10 ou de tout autre moyen adapté.

Par ailleurs, afin de retenir l'embout 10 en position à l'intérieur du flacon à perforer 3, celui-ci peut comprendre, dans une zone intermédiaire s'étendant entre l'orifice proximal 11 et le premier conduit 21 de l'embase 20, des moyens de retenue, de préférence dans une zone adjacente à l'orifice proximal 11 afin que celui-ci reste en permanence à l'intérieur du flacon 3.

Par exemple, les moyens de retenue peuvent comprendre des ailettes 13, présentant une extrémité proximale divergente formant un épaulement 13a adapté pour venir en butée contre le bouchon 3a du flacon 3 et ainsi empêcher l'extraction du dispositif d'interfaçage 1. Par ailleurs, les ailettes 13 peuvent en outre comprendre une extrémité distale convergente 13b facilitant la pénétration de l'embout 10 dans le flacon 3. Afin de limiter la détérioration du bouchon 3a, l'embout 10 comprend de préférence entre deux et quatre ailettes 13.

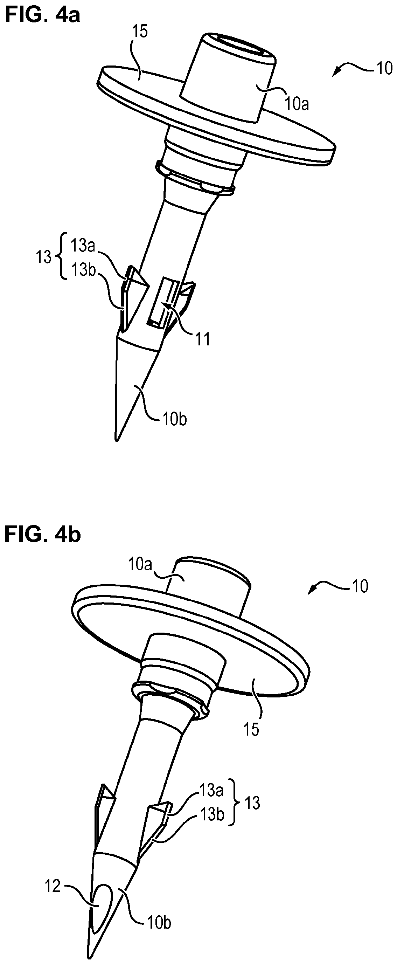

Dans la forme de réalisation illustrée sur les figures, l'orifice proximal 11 est formé entre deux des ailettes 13. Ainsi, même lorsque l'épaulement 13a vient en butée contre le bouchon 3a, la communication fluidique entre le flacon 3 et l'orifice proximal 11 est maintenue (figures 4a, 4b).

Afin de faciliter l'aspiration du gaz et du fluide, l'opérateur peut notamment faire pivoter l'embout 10 dans le bouchon 3a du flacon à perforer 3. La liaison entre l'embout 10 et le bouchon 3a du flacon à perforer 3 est en effet du type rotule de par la souplesse du bouchon 3a et la forme de l'embout 10, et permet donc à l'opérateur d'accéder à l'ensemble du volume interne du flacon 3.

L'extrémité proximale de l'embout 10 comprend un disque 15 s'étendant transversalement à l'axe X1 et configuré pour s'étendre en dehors du flacon à perforer 3 pour faciliter la préhension du dispositif d'interfaçage 1, en particulier lors de l'insertion S2 de l'embout 10 à travers le bouchon à perforer 3a.

L'embout 10 peut être formé intégralement et en une seule pièce, notamment en plastique, avec le premier conduit 21 de l'embase 20, ou en variante être rapporté sur l'extrémité distale 21b du premier conduit 21 puis rendu solidaire de celui-ci, par collage, soudage, ou tout autre moyen adapté.

Selon une variante de réalisation (non illustrée sur les figures), à la fois l'orifice proximal 11 et l'orifice distal 42 sont formés dans l'embout 10. L'embout 10 est alors configuré de sorte que, lorsque l'embase 20 est fixée sur le flacon à perforer 3, les deux orifices 11, 40b se situent dans le volume interne du flacon à perforer 3. Dans cette variante de réalisation, l'embout 10 comprend alors un canal dédié pour les gaz, qui débouche au niveau de l'orifice distal 42, et un canal dédié pour les fluides, qui débouche au niveau de l'orifice proximal 11.

En variante (non illustrée sur les figures), l'orifice proximal 11 et l'orifice distal 42 pourraient être formés dans deux canaux distincts et séparés, fixés chacun à l'embase 20 et en communication fluidique avec le conduit correspondant de ladite embase 20.

### Le dispositif d'échange des gaz

Le dispositif d'interfaçage 1 peut notamment comprendre un dispositif d'échange des gaz 30, tel qu'un réservoir 31 ou un filtre, connecté à l'extrémité proximale 22a du deuxième conduit 22 par l'intermédiaire d'une embase de fixation 32. En utilisation, le dispositif d'échange des gaz 30 est en communication fluidique avec le tube, afin de permettre la circulation des gaz en provenance ou vers le flacon à perforer.

Plus précisément, l'embase de fixation 32 peut comprendre un logement adapté pour recevoir de façon étanche l'extrémité proximale 40a du tube 40 tout en permettant la communication fluidique entre le tube 40 et le dispositif d'échange des gaz 30. L'embase de fixation 32 peut notamment être réalisée dans un matériau plastique.

Le deuxième conduit 22 peut notamment comprendre, au niveau de son extrémité proximale 22a, des moyens d'encliquetage adaptés pour coopérer avec l'embase de fixation 32 et la maintenir en position.

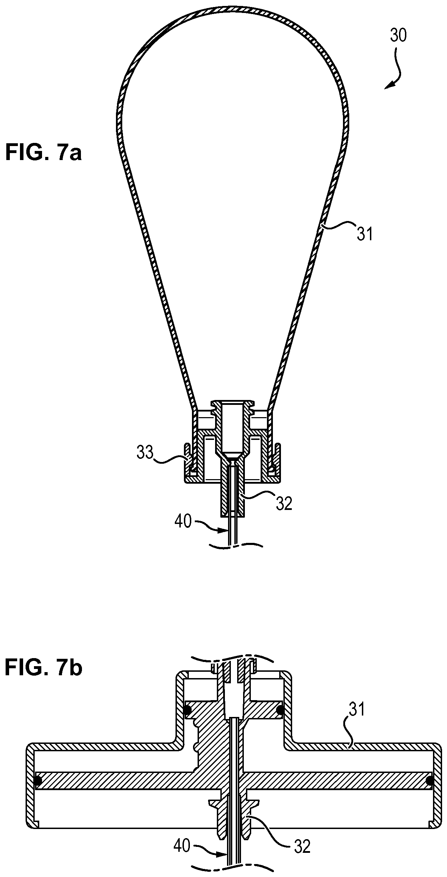

Selon un premier mode de réalisation, le dispositif d'échange des gaz 30 est un réservoir 31 comprenant un ballon élastomérique (figure 7a), fixé à l'embase de fixation 32 par une bague de maintien 33. On pourra notamment se référer au document FR 2 982 484 au nom de la Demanderesse pour plus de détails sur ce type de réservoir. Dans ce mode de réalisation, le ballon 31, l'embase de fixation 32 et la bague de maintien 33 sont solidaires du tube 40, par exemple par collage. De préférence, l'axe principal du ballon 31 s'étend dans le prolongement du tube 40, soit selon l'axe X2 et le cas échéant l'axe X1, pour des raisons d'équilibrage et d'ergonomie (figure 7a).

Selon un deuxième mode de réalisation, le dispositif d'échange des gaz 30 peut comporter un réservoir 31 rigide de volume variable ou carter. Le carter dispose aussi d'une embase de fixation 32 qui relie l'extrémité proximale 40a du tube 40 au réservoir 31 de façon étanche. Ici encore, on pourra notamment se référer au document FR 2 982 484 au nom de la Demanderesse pour plus de détails (figure 7b). Avantageusement, un tel réservoir 31 étant rigide, il ne présente pas le risque d'être accidentellement perforé lors de son stockage ou de sa manipulation par un opérateur.

### L'Organe de protection

Afin d'éviter que l'opérateur ne vienne en contact avec d'éventuelles gouttes du produit contenu dans le flacon à perforer 3, le dispositif d'interfaçage 1 peut comprendre un organe de protection 60, pouvant être fixé sur le flacon à perforer 3 de manière à s'étendre entre le bouchon 3a et l'embase 20.

L'organe de protection 60 peut par exemple comprendre un sachet protecteur, pouvant être fixé d'une part à l'embase 20, et d'autre part au flacon à perforer 3. Pour cela, le sachet protecteur 60 peut par exemple être fixé solidairement au niveau du premier conduit 21 de l'embase 20 et comprendre un moyen élastique de fixation, adapté pour être emmanché sur le bouchon 3a et venir en prise dans une zone sous-jacente de celui-ci.

Des moyens de préhension 61 peuvent être attachés à ce sachet, typiquement dans une zone adjacente au moyen élastique de fixation du sachet 60.

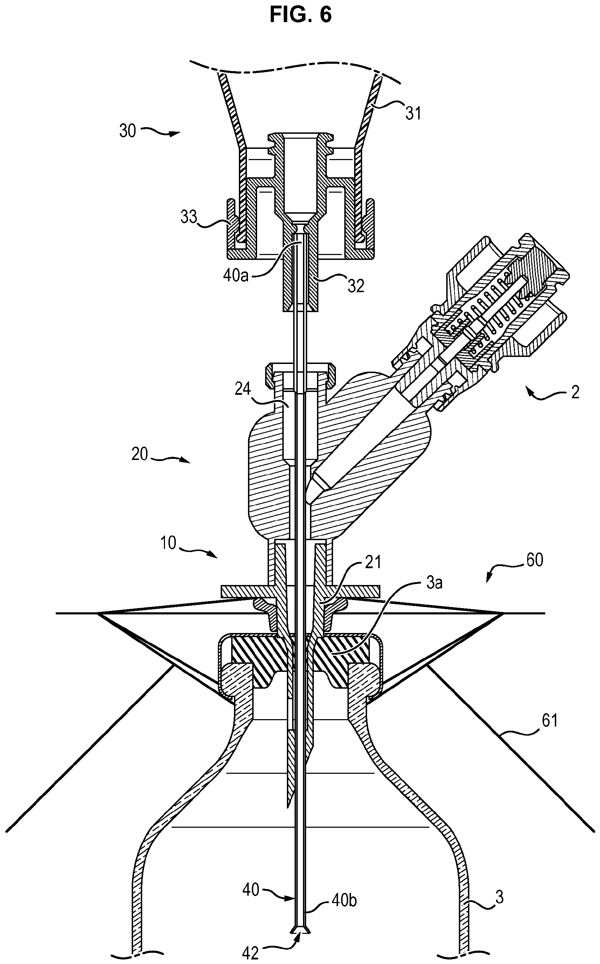

Une fois le dispositif d'interfaçage 1 fixé sur le flacon à perforer 3, le sachet protecteur 60 recouvre donc toute la partie du dispositif d'interfaçage 1 s'étendant entre le bouchon 3a et la zone de l'embase 20 dans laquelle est fixée le sachet 60, empêchant ainsi tout contact entre le contenu du flacon à perforer 3 et l'opérateur (figures 1 et 6).

### Procédé d'utilisation

Nous allons à présent décrire un procédé S d'utilisation d'un dispositif d'interfaçage 1 conforme à l'invention.

Dans ce qui suit, nous décrirons plus particulièrement l'utilisation d'un dispositif d'interfaçage dans lequel les premier 21 et deuxième 22 conduits de l'embase 20 sont sensiblement parallèles et alignés et reçoivent un tube 40 mobile en translation. Par ailleurs, dans ce qui suit, le dispositif d'interfaçage 1 comprend un réservoir 31, sous la forme d'un carter, et l'embout 10 joue le rôle de perforateur.

Ceci n'est cependant pas limitatif car, comme indiqué plus haut, le dispositif d'interfaçage 1 ne comprend pas nécessairement de tube 40 mobile en translation dans les premier 21 et deuxième 22 conduits, ni de réservoir de type carter 31 (qui pourrait tout à fait être remplacé par un filtre ou un ballon élastomérique). Par ailleurs, le rôle du perforateur pourrait être joué par une autre pièce que l'embout 10.

Ici, le flacon à perforer 3 contient un produit sous forme de poudre et du gaz inerte. L'homme du métier saura toutefois facilement adapter ce procédé à un flacon à perforer contenant initialement un fluide ou de la poudre sous vide, la seule différence résidant dans le fait que le flacon à perforer 3 ne comprend pas initialement un gaz devant être évacué dans le réservoir 31.

Au cours d'une première étape S1, l'opérateur chasse le gaz contenu le cas échéant dans le réservoir 31 et connecte le dispositif d'injection de fluide 2 au troisième conduit 23 de l'embase 20, en veillant de préférence à éviter toute formation de goutte pour limiter les risques de contamination, puis l'opérateur injecte le fluide dans le flacon à perforer 3.

Dans le cas où le troisième conduit 23 du dispositif d'interfaçage 1 est étanchéifié à l'aide d'une membrane, on comprendra que la connexion du dispositif d'injection de fluide 2 à l'embase 20 peut être réalisée suite à l'étape S2 de perforation du flacon à perforer 3.

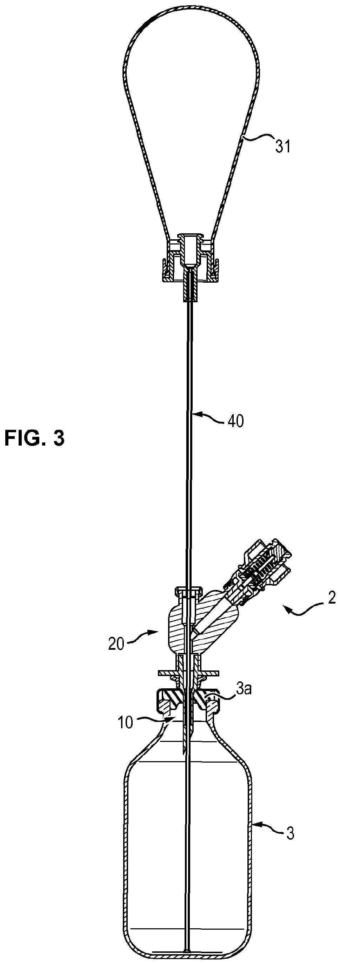

Au cours d'une deuxième étape S2, l'opérateur peut alors perforer le bouchon 3a du flacon 2 en poussant la partie distale 10b pointue de l'embout contre le bouchon 3a. Afin de faciliter cette perforation, le flacon à perforer 3 peut être posé verticalement sur un support, le col vers le haut, et l'embout 10 peut être enfoncé dans le bouchon 3 du haut vers le bas. Pour cette opération, l'extrémité distale 40b du tube 40 est placée de préférence en butée contre l'orifice distal 12 de l'embout 10, afin de ne pas abîmer le tube 40 (figure 3).

Avantageusement, le manchon 24 étanchéifie le deuxième conduit 22 tandis que la membrane étanchéifie le troisième conduit 23, évitant ainsi le passage de gaz chargé le cas échéant de poudre contenue initialement dans le flacon à perforer 3.

Au cours d'une troisième étape S3, l'opérateur injecte du fluide dans le flacon à perforer 3.

L'injection de fluide dans le flacon à perforer 3 a pour effet de chasser le gaz présent initialement dans le flacon à perforer 3 vers le réservoir 31, dont le volume interne augmente alors proportionnellement au volume de gaz reçu.

Au cours d'une quatrième étape S4, l'opérateur peut alors retourner l'ensemble de manière à positionner le dispositif d'injection de fluide 2 verticalement, le flacon à perforer 3 au-dessus du dispositif d'interfaçage 1, de sorte que l'orifice proximal 11 de prise de fluide se trouve immergé (figure 1, sans dispositif d'injection de fluide 2)
Par ailleurs, la configuration de l'embase 20 (premier conduit 21 et deuxième conduit 22 s'étendant dans le prolongement l'un de l'autre, le troisième conduit 23 s'étendant de biais) rend la manipulation du dispositif d'interfaçage 1 plus ergonomique et aisée : il est en effet possible de positionner le dispositif d'interfaçage 1 et le flacon à perforer 3 de telle sorte le troisième conduit 23 s'étende sensiblement verticalement, facilitant ainsi l'aspiration et l'introduction des fluides dans le flacon à perforer 3 à l'aide du dispositif d'injection 2. On rappelle par ailleurs que le mécanisme de remplissage et de purge du réservoir 31 est effectué de façon passive, c'est-à-dire sans intervention de l'opérateur, par simple ajustement de la pression interne du réservoir 31 et du flacon à perforer 3.

Au cours d'une cinquième étape S5, l'opérateur enfoncer le tube 40 dans le flacon à perforer 3 de manière à amener l'orifice distal 42, dans la zone du flacon à perforer 3 contenant les gaz, soit vers le fond de celui-ci.

Ainsi, l'orifice proximal 11 est immergé dans le fluide, tandis que l'orifice distal 42 est amené dans la zone occupée par les gaz, ce qui permet de décorréler efficacement les deux prises de fluide.

Avantageusement, l'effet rotule autorisé par le bouchon 3a permet d'amener l'orifice distal 42 en tout point du flacon à perforer 3.

Au cours d'une sixième étape S6, l'opérateur peut mélanger le liquide et la poudre le contenu du flacon à perforer 3 en aspirant puis réinjectant à plusieurs reprises à l'aide du dispositif d'injection 2 le fluide chargé en produit, de manière à solubiliser le produit initialement sous forme de poudre dans le fluide et à l'homogénéiser. Si besoin, l'opérateur peut faire venir en butée les épaulements 13a contre le bouchon 3a afin de rapprocher encore l'orifice proximal 11 de prise de fluide du bouchon 3a.

Au cours de cette étape S6,
- l'orifice distal 42 reste hors du fluide, ce qui permet d'éviter la création de bulles dans le fluide ou de perdre du fluide dans le réservoir 31 par l'intermédiaire tube 40,
- l'orifice proximal 11 se situe à proximité du bouchon 3a du flacon à perforer 3, ce qui permet de prélever sensiblement tout le fluide.

Cette position retournée est plus ergonomique pour l'opérateur et moins fatigante, en particulier lorsque le fluide est visqueux. Le centre de gravité du système est en effet assez proche de l'axe du dispositif d'injection de fluide 2. L'ergonomie et la praticité de cette position retournée sont rendues possibles grâce à l'angle α entre l'axe X2 du deuxième conduit 22 et l'axe X3 du troisième conduit 23 qui est préférablement compris entre 30 et 60°. En effet, on profite ainsi de la gravité pour aspirer le fluide plus facilement.

En outre, le dispositif d'injection de fluide 2 est rendu solidaire du flacon à perforer 3 par le biais de l'embout 10 et de l'embase 20, ce qui simplifie grandement sa manipulation.

Alternativement, l'opérateur peut retourner à nouveau le dispositif d'interfaçage 1 pour réinjecter le fluide dans le flacon à perforer 3, pour profiter ainsi à nouveau de la gravité. Pour cela, l'opérateur peut aussi refaire venir le tube 40 en butée contre l'extrémité distal 10b l'embout 10. Concrètement, si le besoin s'en fait ressentir au niveau de la manipulation, l'opérateur peut utiliser le dispositif d'interfaçage 1 en position normale, c'est-à-dire flacon à perforer 3 en bas à chaque fois qu'il souhaite injecter du liquide dans le flacon à perforer 3 puis en position retournée, c'est-à-dire flacon à perforer 3 en haut et tube 40 en butée contre le fond dudit flacon 3, à chaque fois qu'il souhaite aspirer du liquide du flacon à perforer 3.

Grâce au sachet 60 formant l'organe de protection, l'opérateur est protégé des éventuelles gouttes qui pourraient fuir entre l'embout 10 et le bouchon 3a, notamment en cas de bouchon 3a de mauvaise qualité ou détérioré, ou d'une mauvaise manipulation du dispositif d'interfaçage 1.

Puis, après avoir dilué le produit de façon satisfaisante, au cours d'une étape S7, l'opérateur peut aspirer le volume de fluide désiré dans cette position retournée, puis, après avoir remis le système en position normale, détacher le dispositif d'injection de fluide 2 du dispositif d'interfaçage 1.

Naturellement, la présente invention n'est nullement limitée aux formes de réalisation décrites ci-dessus et représentées sur les dessins, mais l'homme du métier saura y apporter de nombreuses variantes et modifications.

## Revendications

1. Dispositif d'interfaçage (1) destiné à mettre en liaison un instrument d'injection d'un fluide (2) et un flacon à perforer (3), comprenant :
- une embase (20) comprenant :
* un premier conduit (21), adapté pour être fixé au flacon à perforer (3),
* un deuxième conduit (22), adapté pour permettre la circulation des gaz dans le flacon à perforer (3), et
* un troisième conduit (23), adapté pour être connecté à l'instrument d'injection du fluide (2),
- des moyens de circulation des fluides (10, 40), comprenant un orifice proximal (11) en communication fluidique avec le troisième conduit (23),
le dispositif d'interfaçage (1) étant **caractérisé en ce que** les moyens de circulation des fluides (10, 40) comprennent en outre un tube (40), logé dans le deuxième conduit (22) et mobile en translation dans ledit deuxième conduit (22), et comprenant au niveau de son extrémité distale (40b) un orifice distal (42) en communication fluidique avec le deuxième conduit (22), l'orifice proximal (11) et l'orifice distal (42) étant configurés pour déboucher dans le flacon à perforer (3) lorsque le dispositif d'interfaçage (1) est fixé audit flacon à perforer (3).

2. Dispositif selon la revendication 1, dans lequel l'extrémité distale (40b) du tube (40) est évasée.

3. Dispositif selon l'une des revendications 1 ou 2, dans lequel les moyens de circulation des fluides (10, 40) comprennent un embout (10), fixé au niveau d'une extrémité distale (21b) du premier conduit (21) de l'embase, ledit embout (10) comprenant au niveau de son extrémité proximale (10a) l'orifice proximal (11).

4. Dispositif selon les revendications 1 et 3 prises en combinaison, dans lequel le tube (40) est logé dans l'embout (10) et est mobile en translation dans ledit embout (10).

5. Dispositif selon l'une des revendications 3 ou 4, dans lequel l'orifice proximal (11) est formé au niveau d'une extrémité proximale (10a) de l'embout (10).

6. Dispositif selon l'une des revendications 3 à 5, dans lequel une extrémité distale (10b) de l'embout (10) est pointue.

7. Dispositif selon l'une des revendications 1 à 6, dans lequel l'orifice proximal (11) s'étend latéralement par rapport à une direction d'extension (X1, X2) du tube (40).

8. Dispositif selon l'une des revendications 1 à 7, dans lequel une extrémité proximale (40a) du tube (40) est fixée sur un dispositif d'échange des gaz (30).

9. Dispositif selon l'une des revendications 1 à 8, comprenant en outre un manchon (24) disposé dans le deuxième conduit (22) et adapté pour maintenir le tube (40) de façon étanche dans ledit deuxième conduit (22).

10. Dispositif selon l'une des revendications 1 à 9, comprenant en outre un organe de protection (60), fixé sur le premier conduit (21), et adapté pour être fixé au flacon à perforer (3).

11. Dispositif selon l'une des revendications 1 à 10, dans lequel le premier conduit (21) et le deuxième conduit (22) sont coaxiaux, le troisième conduit (23) formant un angle (α) compris entre 30° et 60° avec le deuxième conduit (23).

## Patentansprüche

1. Vorrichtung zur Schnittstellenbildung (1) zum Verbinden eines Fluidinjektionsinstruments (2) und einer zu durchstechenden Ampulle (3), umfassend:
- einen Sockel (20) umfassend:
* eine erste Leitung (21), die dazu ausgelegt ist, an der zu durchstechenden Ampulle befestigt zu werden (3),
* eine zweite Leitung (22), die dazu ausgelegt ist, das Strömen von Gasen in die zu durchstechende Ampulle (3) zu ermöglichen, und
* eine dritte Leitung (23), die dazu ausgelegt ist, an das Fluidinjektionsinstrument (2) angeschlossen zu werden,
- Fluidströmungsmittel (10, 40), umfassend eine proximale Öffnung (11), die in Fluidkommunikation mit der dritten Leitung (23) steht,
wobei die Vorrichtung zur Schnittstellenbildung (1) **dadurch gekennzeichnet ist, dass** die Fluidströmungsmittel (10, 40) ferner ein Rohr (40) umfassen, das in der zweiten Leitung (22) aufgenommen ist und in der zweiten Leitung (22) translatorisch beweglich ist und an seinem distalen Ende (40b) eine distale Öffnung (42) umfasst, die in Fluidkommunikation mit der zweiten Leitung (22) steht, wobei die proximale Öffnung (11) und die distale Öffnung (42) so konfiguriert sind, dass sie in die zu durchstechende Ampulle (3) münden, wenn die Vorrichtung zur Schnittstellenbildung (1) an der zu durchstechenden Ampulle (3) befestigt ist.

2. Vorrichtung nach Anspruch 1, bei der das distale Ende (40b) des Rohrs (40) aufgeweitet ist.

3. Vorrichtung nach einem der Ansprüche 1 oder 2, bei der die Fluidströmungsmittel (10,40) ferner ein Mundstück (10) umfassen, das an einem distalen Ende (21b) der ersten Leitung (21) des Sockels befestigt ist, wobei das Mundstück (10) an seinem proximalen Ende (10a) die proximale Öffnung (11) umfasst.

4. Vorrichtung nach den Ansprüchen 1 und 3 in Kombination, bei der das Rohr (40) in das Mundstück (10) aufgenommen ist und in dem Mundstück (10) translatorisch beweglich ist.

5. Vorrichtung nach einem der Ansprüche 3 oder 4, bei der die proximale Öffnung (11) an einem proximalen Ende (10a) des Mundstücks (10) ausgebildet ist.

6. Vorrichtung nach einem der Ansprüche 3 bis 5, bei der ein distales Ende (10b) des Mundstücks (10) spitz ist.

7. Vorrichtung nach einem der Ansprüche 1 bis 6, bei der sich die proximale Öffnung (11) seitlich in Bezug auf eine Ausdehnungsrichtung (X1, X2) des Rohrs (40) erstreckt.

8. Vorrichtung nach einem der Ansprüche 1 bis 7, bei der ein proximales Ende (40a) des Rohrs (40) an einer Gasaustauschvorrichtung (30) befestigt ist.

9. Vorrichtung nach einem der Ansprüche 1 bis 8, ferner umfassend eine Hülse (24), die in der zweiten Leitung (22) angebracht und dazu ausgelegt ist, das Rohr (40) abdichtend in der zweiten Leitung (22) zu halten.

10. Vorrichtung nach einem der Ansprüche 1 bis 9, ferner umfassend ein Schutzelement (60), das an der ersten Leitung (21) befestigt ist und dazu ausgelegt ist, an der zu durchstechenden Ampulle (3) befestigt zu werden.

11. Vorrichtung nach einem der Ansprüche 1 bis 10, bei der die erste Leitung (21) und die zweite Leitung (22) koaxial sind, wobei die dritte Leitung (23) mit der zweiten Leitung (23) einen Winkel (α) zwischen 30° und 60° bildet.

## Claims

1. Device for interfacing (1) for establishing a connection between a fluid-injection instrument (2) and a flask to be perforated (3), comprising:
- a base (20) comprising:
* a first duct (21), suitable for being attached to the flask to be perforated (3),
* a second duct (22), suitable for allowing gas flow circulation in the flask to be perforated (3), and
* a third duct (23), suitable for being connected to the fluid-injection instrument (2),
- fluid flow circulation means (10, 40), comprising a proximal opening (11) in fluidic communication with the third duct (23),
The interfacing device (1) being **characterised in that** the fluid flow circulation means (10, 40) further comprise a tube (40), housed in the second duct (22) and translatable in said second duct (22), and comprising at its distal end (40b) a distal opening (42) in fluid communication with the second duct (22), the proximal opening (11) and the distal opening (42) being configured such as to open into the flask to be perforated (3) when the interfacing device (1) is attached to said flask to be perforated (3).

2. Device according to claim 1, wherein the distal end (40b) of the tube (40) is flared.

3. Device according to one of claims 1 or 2, wherein the fluid flow circulation means (10, 40) include a tip (10), attached at a distal end (21b) of the first duct (21) of the base, said tip (10) comprising at its proximal end (10a) the proximal opening (11).

4. Device according to claims 1 and 3 taken in combination, wherein the tube (40) is housed in the tip (10) and is translatable within said tip (10).

5. Device according to one of claims 3 or 4, wherein the proximal opening (11) is formed at a proximal end (10a) of the tip (10).

6. Device according to one of claims 3 to 5, wherein a distal end (10b) of the tip (10) is pointed.

7. Device according to one of claims 1 to 6, wherein the proximal opening (11) extends laterally with respect to a direction of extension (X1, X2) of the tube (40).

8. Device according to one of claims 1 to 7, wherein a proximal end (40a) of the tube (40) is attached on an interfacing device of the gases (30).

9. Device according to one of claims 1 to 8, further comprising a sleeve (24) arranged in the second duct (22) and adapted to maintain the tube (40) in a sealed manner in said second duct (22).

10. Device according to one of claims 1 to 9, further comprising a protective member (60), attached on the first duct (21), and suitable for being attached to the flask to be perforated (3).

11. Device according to one of claims 1 to 10, wherein the first duct (21) and the second duct (22) are coaxial, the third duct (23) forming an angle (α) between 30° and 60° with the second duct (23).
